# EUROPEAN PATENT SPECIFICATION

(11) **EP 2 007 370 B1**
(45) Date of publication and mention of the grant of the patent: **18.12.2013**
(21) Application number: 07747360.1
(22) Date of filing: 02.04.2007
(51) Int. Cl.: A61K 31/165, A61K 31/336, A61K 38/22, A61K 38/31, A61K 38/00, A61K 31/48, A61P 35/00, A61P 19/02

(54) **NOVEL COMPOSITION FOR TUMOR GROWTH CONTROL**
NEUE ZUSAMMENSETZUNG ZUR KONTROLLE DES TUMORWACHSTUMS
NOUVELLE COMPOSITION POUR LE CONTROLE DE LA CROISSANCE TUMORALE

(30) Priority: 31.03.2006 WO PCT/NL2006/000165
(43) Date of publication of application: 31.12.2008
(60) Divisional application: 11168542.6
(73) Proprietor: Erasmus University Medical Center Rotterdam, 3015 GE Rotterdam (NL)
(72) Inventor: HOFLAND, Leendert, Johannes, NL-3015 GE Rotterdam (NL)
(74) Representative: Wells, Andrew
(86) International application number: PCT/NL2007/050136
(87) International publication number: WO 2007/114697

(56) References cited:
- EP-A- 1 477 165
- EP-A- 1 547 617
- EP-A2- 0 266 665
- WO-A-00/75186
- WO-A-98/16208
- WO-A-02/067681
- WO-A-2005/023179
- WO-A2-2005/039498
- WO-A2-2005/066151
- GB-A- 2 351 733
- US-A- 5 538 739
- US-A1- 2004 109 846
- US-A1- 2005 148 500
- US-A1- 2006 069 060
- MCNICHOL A M ET AL: "Clinical and histological correlations in prolactinomas, with special reference to bromocriptine resistance - Comments" ACTA NEUROCHIRURGICA, vol. 147, no. 7, July 2005 (2005-07), pages 757-758, XP002411478 ISSN: 0001-6268
- EYAL ORI ET AL: "A case of macroprolactinoma and elevated insulin-like growth factor-I in a young boy" ACTA PAEDIATRICA, vol. 94, no. 12, December 2005 (2005-12), pages 1852-1854, XP009076130 ISSN: 0803-5253
- PÉREZ-ALVAREZ R ET AL: "Sarcoidosis in two patients with chronic hepatitis C treated with interferon, ribavirin and amantadine." JOURNAL OF VIRAL HEPATITIS. JAN 2002, vol. 9, no. 1, January 2002 (2002-01), pages 75-79, XP002411479 ISSN: 1352-0504
- JAQUET P ET AL: "Efficacy of chimeric molecules directed towards multiple somatostatin and dopamine receptors on inhibition of GH and prolactin secretion from GH-secreting pituitary adenomas classified as partially responsive to somatostatin analog therapy" EUROPEAN JOURNAL OF ENDOCRINOLOGY, vol. 153, no. 1, July 2005 (2005-07), pages 135-141, XP002440751 ISSN: 0804-4643
- ZATELLI MARIA CHIARA ET AL: "Evidence for differential effects of selective somatostatin receptor subtype agonists on alpha-subunit and chromogranin A secretion and on cell viability in human nonfunctioning pituitary adenomas in vitro" JOURNAL OF CLINICAL ENDOCRINOLOGY & METABOLISM, vol. 89, no. 10, October 2004 (2004-10), pages 5181-5188, 5175, XP002440752 ISSN: 0021-972X
- DONANGELO, INES; MELMED, SHLOMO: "Treatment of acromegaly" ENDOCRINE, vol. 28, no. 1, 2005, pages 123-128, XP009085929
- DASGUPTA P: "GH-inhibitory activity of novel somatostatin agonists: Potential applications in acromegaly" CURRENT MEDICINAL CHEMISTRY - IMMUNOLOGY ENDOCRINE & METABOLIC AGENTS, vol. 5, no. 3, June 2005 (2005-06), pages 277-291, XP009085930 ISSN: 1568-0134
- GRUSZKA ET AL: "The effect of selective sst1, sst2, sst5 somatostatin receptors agonists, a somatostatin/dopamine (SST/DA) chimera and bromocriptine on the ''clinically non-functioning'' pituitary adenomas in vitro" LIFE SCIENCES, PERGAMON PRESS, OXFORD, GB, vol. 78, no. 7, 11 January 2006 (2006-01-11), pages 689-693, XP005221165 ISSN: 0024-3205

## Description

The invention relates to the field of cancer, more specifically to treatment of tumors, more specifically to the treatment of tumors which express a somatostatin and/or dopamine receptor.

In mammalian cells, approximately 35 to 5% of the cytosine residues in genomic DNA are present as 5-methylcytosine (Ehrlich et al., 1982, Nucl. Acids Res. 10:2709-2721). This modification of cytosine takes place after DNA replication and is catalyzed by DNA methyltransferase using S-adenosyl-methionine as the methyl donor. Approximately 70% to 80% of 5-methylcytosine residues are found in the CpG sequence (Bird, 1986, Nature 321:209-213). This sequence, when found at high frequency in the genome, is referred to as CpG islands. Unmethylated CpG islands are associated with housekeeping genes, while the islands of many tissue-specific genes are methylated, except in the tissue where they are expressed (Yevin and Razin, 1993, in DNA Methylation: Molecular Biology and Biological Significance. Birkhauer Verlag, Basel, p. 523-568). This methylation of DNA has been proposed to play an important role in the control of expression of different genes in eukaryotic cells during embryonic development. Consistent with this hypothesis, inhibition of DNA methylation has been found to induce differentiation in mammalian cells (Jones and Taylor, 1980, Cell 20:85-93).

Methylation of DNA in the regulatory region of a gene can inhibit transcription of the gene. This is probably caused by intrusion of the 5-methylcytosine into the major groove of the DNA helix, which interferes with the binding of transcription factors.

Three major changes in DNA methylation commonly occur in cancer compared to normal tissue. Firstly, cancer cells show genome wide hypomethylation, which has been associated with chromosomal instabilities (Eden et al., 2003, Science 300:455; Gaudet et al., 2003, Science 300:489-492), as well as activation of normally silenced repetitive DNA elements (Walsh et al., 1998, Nat. Genet. 20, 116-117).

Secondly, de novo methylation of CpG islands can occur throughout tumor development. It is estimated that in tumors there are on average 600 CpG islands aberrantly methylated compared to normal tissue, although this can vary widely between tumour types and within particular histological subtypes. Moreover, methylation does not occur randomly, as there are CpG islands that are methylated in multiple tumour types, while other CpG islands are only methylated in certain tumour types (Costello, J.F., et al., 2000, Nat. Genet. 24:132-138; Esteller, M., Herman, J.G., 2002, J. Pathol. 196:1-7). This is consistent with a model in which methylation of CpG islands at particular genes would give the cancer cell a growth or survival advantage and so patterns of methylation emerge depending on the selective pressure for gene silencing in the tumour type examined.

Thirdly, the methylated cytosine in DNA, 5-methylcytosine, can undergo spontaneous deamination to form thymine at a rate much higher than the deamination of cytosine to uracil (Shen et al., 1994, Nucl. Acids Res. 22:972-976). If the deamination of 5-methylcytosine is unrepaired, it will result in a C to T transition mutation. This can be found in the coding region, but many tumor suppressor genes can also be inactivated by aberrant methylation of the CpG islands in their promoter regions. The molecular mechanisms by which aberrant methylation of DNA takes place during tumorigenesis are not clear. It is possible that the DNA methyltransferase makes mistakes by methylating CpG islands in the nascent strand of DNA without a complementary CpG in the parental strand. It is also possible that aberrant methylation may be due to the removal of CpG binding proteins that protect these sites from being methylated. Whatever the mechanism, the frequency of aberrant methylation is a rare event in normal mammalian cells.

On basis of the above, compounds that inhibit methylation, such as derivatives of 2'-deoxycitidine (5-aza-2'-deoxycitidine (DAC, Decitabine), 5-azacytidine (5-azaC), arabinosyl-5-azacytosine (fazarabine) and dihydro-5-azacytidine (DHAC)) have been proposed to have anti-tumor properties, since they can activate the expression of epigenetically silenced genes including tumor suppressing genes. In addition, these demethylating agents can restore sensitivity to a range of chemotherapeutic agents including cisplatin, epirubicin and temolozomide (Plumb, J.A. et al., 2000, Cancer Res. 60:6039-6044; Strathdee, G. et al., 1999, Oncogene 18:2335-2341). Like many other novel therapeutics currently being developed against specific targets, demethylating agents are anticipated to inhibit tumor growth by specifically reversing the repression of tumor suppressor and cell cycle genes that are aberrantly methylated in tumor cells, and hence to have less side effects than non-specific conventional chemotherapy (Issa, J.P., 2003, Curr. Opin. Oncol. 15:446-451). Synergistic cytotoxicity of Decitabine and a variety of clinically important cytotoxic agents has been reported *in vitro* and *in vivo*, including amsacrine, cisplatin, carboplatin, cyclophosphamide, cytarabine, daunorubicin, epirubicin, idarubicin, interpheron alpha, retinoic acid, topotecan and temozolomide (Frost, P. et al., 1990, Cancer Res. 50:4572-4577;. Kritz et al., 1996, Am. J. Hematol. 51(2):abstract; Colombo et al., 1986 Cancer Treat. Rep. 70(12): abstract; Momparler, R.L. et al., 1990, Cancer Lett. 54:21-28; Anzai et al., 1992, Cancer Res. 52(8): abstract; Dore et al., 1992, Anticancer Drugs 3(3): abstract; Schwartsmann et al., 1997, Leukemia 11(Suppl. 1): abstract; Willemze et al., 1997, Leukemia 11(Suppl. 1): abstract; Plumb et al., *supra*). A combination of demethylating agents and all known anti-tumor pharmaceuticals has been claimed, but not demonstrated, by Rubinfeld, J. et al. (WO 2002/067681).

Epigenetic silencing can also be inhibited by blocking histone deacetylases (HDACs) through histone deactylation inhibitors (HDAC inhibitors). Next to methylation of cytosine residues in DNA, as described above, removal of acetyl groups is a primary mechanism for gene silencing.

The deposition of histones throughout the cell cycle by a replication-independent process implies that previously existing nucleosomes are unraveled, and their histones released. It is known that the process of transcription results in a local unfolding of the chromatin fiber and an 'open' chromatin configuration. Although transcription of nucleosomal templates with bacterial polymerases can occur in vitro without displacing histone octamers from DNA, in vivo assays demonstrated that a measurable amount of transcription-dependent histone displacement does occur in eukaryotic nuclei. In fact, even in vitro, RNA polymerase II is virtually unable to transcribe nucleosomal DNA under physiological conditions. Transcription requires that histone-DNA contacts be broken for polymerase to transit the nucleosomal DNA. Although transcription can occur without histone displacement if the histone octamer releases some contacts with DNA and maintains others, at some frequency all contacts might be released. The histone octamer would then simply fall off. Additionally, localized remodeling factors will disrupt nucleosome structure as they act. The in vitro and in vivo observations can be reconciled if histone displacement occurs occasionally as nucleosomes are disrupted. Constraints on nucleosomes in a compacted chromatin fiber (i.e., 'closed' chromatin) would limit histone displacement.

Hypoacetylation, particularly of histones H3 and H4, associated with heterochromatic domains from a range of organisms, has been studied in greatest detail in *Saccharomyces cerevisiae.* Many of the cis- and trans-acting factors necessary to establish and maintain the silent state at the telomeres and HML/HMR loci have been identified. These studies have demonstrated the need for hypoacetylated histones. Silencing is mediated by the multiprotein, nucleosome binding SIR₍₁₋₄₎ complex, recruited by interaction with specific DNA binding proteins. Sir3 and Sir4 interact specifically with the N-terminal tails of histones H3 and H4 in the hypoacetylated state. While the N-terminal tails of the histones are not required individually for growth in yeast, they do play an essential role in silencing, amino acids 4-20 of H3 and 16-29 of H4 being required. Certain sir3 alleles can suppress the silencing defect of histone H4 tail mutations, and Sir3 and Sir4 can bind to the amino termini of histones H3 and H4 in vitro, suggesting direct interaction. Recent studies using antibodies against different histone acetylated isoforms indicate that histones in the telomeric and HML/HMR heterochromatin are hypoacetylated at all modification sites

HDAC inhibitors act on histone acetylation during the first 24 hr after addition of the drug to the cell culture, usually peaking within the first 16 hr (the exact time depends on the cell line, the compound, etc.). This effect may be reversed by growing the cells in fresh medium with no drug. Changes in the gene expression profile also appear during the first 24 hr of treatment, when histones become hyperacetylated. All the known HDAC inhibitors cause an increase at both the mRNA and protein levels of the cyclin-dependent kinase inhibitor p21WAF1 as well as cell-cycle arrest. Although they act by similar mechanisms (e.g., by blocking access of acetyl-lysine residues to the HDAC catalytic pocket, which is highly conserved in all HDACs), these substances have markedly different effects: diverse patterns of gene expression are found when observing the action of several compounds in the same cell line and cell-cycle arrest can be produced at G2/M (most frequently) or at G/S.

In neuroendocrine tumors, as well as in undifferentiated epithelial tumors, such as breast, prostate and colon carcinomas, somatostatin and/or dopamine receptors are expressed at the tumor cell surface (Reubi, J.C. and Landolt, A.M., 1989, J. Clin. Endocrinol. Metab. 68:844-850; Chanson, P. et al., 1993, Ann. Intern. Med. 119:236-240; De Bruin, T.W.A., 1992, J. Clin. Endocrinol. Met. 75:1310-1317; De Herder, W.W., 1994, Am. J. Med. 96:305-312; Reubi, J.C. et al., 1990, Metabolism 39(Suppl. 2):78-81; Lamberts, S.W.J., 1991, Endocr. Rev. 12:450-482; Lemmer, K. et al., 2002, Life Sci. 71:667-678). In patients with somatostatin and/or dopamine receptor positive tumors, these G-protein coupled receptors would form a target for treatment with somatostatin analogs and/or dopamine agonists, respectively, since these would inhibit the production of hormones and bioactive peptides by the tumor cells, resulting in a better control of the related severe clinical symptomatology. While somatostatin analogs and dopamine agonists inhibit tumor cell growth in a number of experimental models (Lamberts et al., 1991, *supra*; Schally, A.V., 1988, Cancer Res. 48:6977-6985; Lamberts, S.J.W. et al., 1996, New. Eng. J. Med. 334:246-254), such a tumor growth inhibitory effect is not, or only rarely observed clinically (Hejna, M. et al. , 2002, "The clinical role of somatostatin analogues as antineoplastic agents: much ado about nothing?" Annals of Oncology 13: 653-668) If there is any effect, a dopamine agonist mostly causes tumor regression by inhibition of protein synthesis and decreasing of the cell volume (McNichol, A.M. et al., 2005, Acta Neurochir. 147(7):2005-2007). For somatostatin analogs regression of growth-hormone producing tumors in the hypophyseal gland has been described (WO 00/75186; US 5,538,739), but cessation of treatment, however, often leads to a rapid increase in tumor volume, thus denying a potential growth inhibiting effect. Several possible reasons have been provided why somatostatin analogs appear not to be effective in humans (Lamberts, S.W.J. et al., 2002, Trends Endocrinol. Metabol. 13:451-457): (i) most human cancers comprise a mixture of stromal tissue and different clones of epithelial tumor cells that do not uniformly express somatostatin receptors (sst). This contrasts sharply with the most monoclonal tumor models in animals, which, in most instances, homogeneously express sst on all tumor cells; (ii) sst expression in parts of human breast, prostate and colonic cancers often indicates loss of differentiation of the tumors. In general, these undifferentiated tumors with neuroendocrinologic cell differentiation have a poor prognosis at that stage of development; (3) because of the nature of new clinical trials in oncology, often it is mainly those patients who are late in the onset of their disease who are included in the studies. In addition, there is preliminary evidence that previous chemotherapy might decrease the number of sst; and (iv) it remains unclear whether the sst₂-specific analogs applied in clinical trials so far are the best compounds to use.

US 2005/148500 A1 is concerned with proteinaceous compositions for increasing the levels of dopamine in neurons, and with methods for treating conditions caused by deficiency of dopamine and/or loss of DA neurons or injury to DA neurons.

WO 02/067681 A1 is concerned with compositions and methods for treating diseases associated with abnormal cell proliferation such as cancer, the methods comprising use of a DNA methylation inhibitor in combination with an anti-neoplastic agent whose activity as an anti-neoplastic agent in vivo is adversely affected by aberrant DNA methylation.

WO 00/75186 A1 is concerned with neuromedin B and somatostatin receptor agonists.

US 2006/069060 A1 is concerned with salts of decitabine, methods for synthesising the salts, and uses thereof.

US-A-5,538,739 is concerned with microparticles comprising octreotride, in a specific polymeric matrix.

McNichol A M et al (2005) "Clinical and histological correlations in prolactinomas - Comments" Acta Neurochirurgica 147: 757-758 is concerned with clinicopathological correlations in a series of prolactinomas, examining the relationships between histological signs of aggressiveness and clinical evidence of invasion or resistance to the dopamine agonist, bromocriptine (BRC).

Eyal O et al (2005) Acta Paediatrica 94: 1852-1854 reports a case of macroprolactinoma and elevated insulin-like growth factor-I in a young boy.

Perez-Alvarez R et al (2005) Journal of Viral Hepatitis 9: 75-79 presents a case report of sarcoidosis in two patients with chronic hepatitis C treated with interferon, ribavirin and amantadine.

EP 1,477,165 A2 is concerned with the use of a tetrahydrobiopterin synthesis antagonist for treating inflammation in particular contact dermatitis, rheumatoid arthritis and allograft rejection caused by overproduction of nitric oxide in immune cells.

EP 0,266,665 A2 is concerned with treatment of inflammatory articular diseases using somatostatin.

EP 1,547,617 A1 is concerned with an agent for inhibiting articular cartilage extracellular matrix degradation, comprising a histone deacetylase-inhibiting compound, and the use thereof in treating diseases or conditions involving degradation and degeneration of the articular cartilage extracellular matrix.

US 2004/109846 A1 is concerned with compositions and methods for treating diseases associated with abnormal cell proliferation such as cancer, the methods comprising use of a DNA methylation inhibitor in combination with an anti-neoplastic agent whose activity as an anti-neoplastic agent in vivo is adversely affected by aberrant DNA methylation.

GB-A-2,351,733 is concerned with non-peptide somatostatin agonists, and the use thereof in therapy.

WO 2005/039498 A2 is concerned with methods of treating cancers using histone deacetylase (HDAC) inhibitors.

WO 2005/066151 A2 is concerned with particular histone deacetylase (HDAC) inhibitors.

WO 98/16208 A is concerned with a composition for treating Parkinsons disease comprising an alkyl ester of levodopa in a solvent which allows transdermal administration of levodopa.

WO 2005/023179 A2 is concerned with a method of treating cancer, by administering histone deacetylase inhibitor or a pharmaceutically acceptable salt or hydrate thereof in a first treatment procedure and a second amount of an anti-cancer agent in a second treatment procedure.

Donangelo, Ines & Melmed, Shlomo (2005) Endocrine 28: 123-128 presents a discussion of medical therapy of GH-secreting adenomas.

Gruszka A et al (2006) Life Sciences 78: 689-693 examines the effect of selective sst1, sst2, sst5 somatostatin receptors agonists, a somatostatin/dopamine (SST/DA) chimera and bromocriptine on "clinically non-functioning" pituitary adenomas in vitro.

### Summary of the Invention

The inventors have now, surprisingly found that a combination of a somatostatin analog, a dopamine agonist or a somatostatin-dopamine chimeric molecule (which targets both a somatostatin and dopamine D2 receptor simultaneously) with a demethylating agent overcomes the problems with the therapeutic effectiveness of single treatments of somatostatin analogs and/or dopamine agonists and that treatment with a combination of a somatostatin analog, a dopamine agonist or a somatostatin-dopamine chimeric molecule with a demethylating agent provides an effective therapy for tumors which express somatostatin and/or dopamine receptors, respectively. Since epigenetic silencing is caused not only by DNA methylation but also by histon deacetylation, comparable synergistic effects of a combination of a HDAC inhibitor with a somatostatin analog and/or dopamine agonist, or with a somatostatin-dopamine chimeric molecule, are envisaged.

The invention therefore comprises a pharmaceutical composition comprising at least one demethylating agent and at least one dopamine agonist wherein the at least one demethylating agent is selected from the group consisting of 5-aza-2'-deoxycitidine (DAC, Decitabine), 5-azacytidine (5-azaC), arabinosyl-5-azacytosine (fazarabine) and dihydro-5-azacytidine (DHAC); and the at least one dopamine agonist is selected from the group consisting of amantadine, bromocriptine, cabergoline, quinagolide, lisuride, pergolide, ropinirole, pramiprexole, rasagiline, BIM23A387, BIM-23A758, BIM-23A760, BIM-232A761 and BIM-23A765.

The pharmaceutical composition may further comprise at least one HDAC inhibitor selected from the group consisting of hydroxaminic acids, carboxylic acids, benzamides, epoxides, FK228, apicidin A, B and C.

The pharmaceutical composition may comprise the at least one demethylating agent, the at least one HDAC inhibitor, at least one somatostatin analog selected from the group consisting of somatostatin-14, somatostatin-28, cortistatin-14, cortistatin-17, cortistatin-29, octreotide, vapreotide, lanreotide, CH275, CH-288, BIM-23056, BIM-23206, BIM-23268, BIM-23926, BIM-23052, BIM-23244, BIM-23A758, BIM-23A760, BIM-232A761, BIM-23A765, L- and D-Tyr(8)CYN 154806, L-779,976, L-803,087, L-817,818, BIM23A387, SOM-320 and KE-108, and the at least one dopamine agonist.

In one aspect the demethylating agent is 5-aza-2'-deoxycytidine and the dopamine agonist is cabergoline. In one aspect, the demethylating agent is 5-aza-2'-deoxycytidine and the at least one dopamine agonist is the somatostatin-dopamine chimera BIM-23A765.

The invention also provides a pharmaceutical composition as defined above for use in the treatment of a tumor which expresses dopamine receptors on its surface.

The invention also provides a pharmaceutical composition comprising 5-aza-2'-deoxycytidine and octreotide, and a pharmaceutical composition comprising 5-aza-2'-deoxycytidine and BIM-23926 or BIM-23206.

The invention further provides a pharmaceutical composition as defined above for use in the treatment of a tumour.

### Description of the Figures

Fig. 1. Panel A shows the effects of AZA (Decitabine), octreotide and a combination of both on cell proliferation in human BON carcinoid tumor cells. **Panel B** shows the effects of these compounds on DNA fragmentation (apoptosis) in BON cells.
**Fig. 2****.** Same as Fig. 1 for AZA and cabergoline.
**Fig. 3****.** Synergistic effect of AZA (5nM) and the somatostatin analogs BIM23926 (sst1-selective) and BIM 23206 (sst5-selective) (both 10 nM) on DNA fragmentation of human BON carcinoid cells. Values represent the mean of 2 independent experiments.
**Fig. 4****.** Synergistic effect of AZA (5 nM) and the somatostatin-dopamin chimera BIM 23A765 (10 nM; sst2+sst5+D2 selective agonist) on DNA fragmentation of human BON carcinoid cells. Values represent the mean of 2 independent experiments.

### Detailed Description of the Invention

Due to the problems as described in the introductory part, many attempts to use somatostatin analogs and/or dopamine agonists for the control of tumor growth and development, have failed or were not as successful as might have been expected. One of the hypotheses that have been tested by the current inventors is that the failure to obtain proper tumor growth control with these compounds was due to hypermethylation of tumor genes, especially the genes involved in the tumor growth inhibitory effect via the activation of somatostatin and/or dopamine receptors. The current inventors therefore have set out experiments as reported in the experimental section and surprisingly observed that combined treatment of a somatostatin analog and/or a dopamine agonist, or a somatostatin-dopamine chimeric molecule with a demethylating agent such as Decitabine dramatically increases the effectivity of the somatostatin analog, dopamine agonist or somatostatin-dopamine chimeric molecule. The observed potent synergistic tumor growth inhibitory effect is somatostatin analog, dopamine agonist or somatostatin-dopamine chimeric molecule. The observed potent synergistic tumor growth inhibitory effect is believed to be at least partly due to an increase in DNA fragmentation (apoptosis).

Several demethylating agents, which can be applied in the present invention, are known in the art. One major group is formed by derivatives of 2'-deoxycitidine, of which 5-aza-2'-deoxycytidine (Decitabine) is currently the most employed. The structural difference between decitabine and the natural nucleoside, deoxycytidine, is the presence of a nitrogen at position 5 of the cytosine ring as compared to the carbon atom at this position. Two isomeric forms of Decitabine can be distinguished, of which the β-anomer is the active form. Decabitine possesses multiple pharmacological characteristics. At a molecular level, it is S-phase dependent for incorporation into DNA. At a cellular level, decitabine can induce cell differentiation and exert hematological toxicity. Despite having a short half life *in vivo*, decitabine has an excellent tissue distribution.

The most prominent function of decitabine is its ability to specifically and potently inhibit DNA methylation. Inside the cell, decitabine is first converted to its active form by deoxycytidine kinase which is primarily synthesized during the S phase of the cell. After conversion to its triphosphate form, decitabine is incorporated into replicating DNA at a rate similar to that of the natural substrate, dCTP (Bouchard and Monparler, 1983, Mol. Pharmacol. 24:109-114). The replacement of 5-methylcytosine with decitabine at a specific site of methylation produces an irreversible inactivation of DNA methyltransferase, presumably due to formation of a covalent bond between the enzyme and decitabine. By specifically inhibiting DNA methyltransferase, the enzyme required for DNA methylation, the aberrant methylation of the tumor genes can be prevented.

The investigators have also found that treatment with a demethylating agent increases the expression of somatostatin and dopamine receptors on the surface of the tumor cells (data not shown).

Naturally occurring and synthetic HDAC inhibitors are now of interest to pharmaceutical companies because of their great potential use against cancer and other human pathologies. The biochemical structure of HDAC inhibitors is extremely heterogeneous, from simple compounds, such as valproate, to more elaborate designs, such as MS-275 (a benzamide). Overall, HDAC inhibitors manifest a wide range of activity against all HDACs, although a few exceptions are known. These compounds can be classified according to their chemical nature and mechanism of inhibition as follows.
1. Hydroxamic acids. This is probably the broadest set of HDAC inhibitors. The general structure of these substances consists of a hydrophobic linker that allows the hydroxamic acid moiety to chelate the cation at the bottom of the HDAC catalytic pocket, while the bulky part of the molecule acts as a cap for the tube. Most of the chemicals in this group are very potent (functioning at nanomolar to micromolar concentrations *in vitro*) but reversible inhibitors of class I/II HDACs. Among these compounds we find TSA (trichostatin A), which is widely used as a reference in research in this field. However, its toxicity to patients and lack of specificity for certain HDACs have motivated the search for other substances. The design of many synthetic drugs has been inspired by TSA structure: from the simplicity of SAHA (suberoylanilide hydroxamic acid) or Scriptaid to the latest drugs developed by pharmaceutical companies such as Novartis (Basel, Switzerland), including NVP-LAQ-82425 and PXD-101 there is huge scope for building-block combinations that have been tested (*in vitro*, *in vivo* and, in some instances, in clinical trials) and shown to have an acceptable degree of success.
2. Carboxylic acids. There are only a few drugs in this group: butanoic, valproic and 4-phenylbutanoic acids. Despite being much less potent than the hydroxamic acids (inhibition occurs *in vitro* at millimolar concentrations and at very high doses in vivo) and their pleiotropic effects, these are currently among the best studied HDAC inhibitors: valproic acid and phenylbutyrate have already been approved for use in treating epilepsy and some cancers, respectively, whereas butanoic acid (or its prodrug forms, such as pivaloyloxymethylbutyrate) is undergoing clinical trials.
3. Benzamides. MS-275 and some of its derivatives inhibit HDACs in vitro at micromolar concentrations, but the mechanism is not clearly understood. It is believed that the diaminophenyl group is very important for the inhibitory behavior; probably, both amino functionalities chelate the metallic ion in the catalytic site. MS-275 and N-acetyldinaline are undergoing clinical trials.
4. Epoxides. These chemicals are supposed to trap HDACs through the reaction of the epoxide moiety with the zinc cation or an amino acid (forming a covalent attachment) in the binding pocket. However, the lability of the epoxide functionality prevents significant *in vivo* activity, which makes them of little pharmacologic interest. The only HDAC inhibitors in this set of compounds are a number of natural products with significant *in vitro* activity, such as depeudecin, trapoxin A, etc. Trapoxins are cyclic tetrapeptides with at least one nonproteinogenic amino acid, and their structures have been used as models for the design of novel hydroxamic acid- based HDAC inhibitors.
5. Others. Like the benzamides in the previous group, depsipeptide FK228 (a fungal metabolite) is also undergoing clinical trials, but the mechanism by which it inhibits classical HDACs *in vitro* remains unknown. One hypothesis proposes that the disulfide bridge is reduced inside the cell or organism and the 4-mercapto-but-1-enyl residue then fits inside the HDAC catalytic pocket, chelating Zn²⁺ in a manner similar to that of other inhibitors. In cultured cells, it is able to induce histone hyperacetylation and growth arrest at nanomolar concentrations. Apicidin A is another fungal metabolite that is able to inhibit HDACs in many organisms, from protozoa to humans, at micromolar concentrations. Chemically, it is a cyclic tetrapeptide bearing an differing from apicidin A by a single residue. Indeed, the peptidic nature of apicidins allows the chemical synthesis (including those employing solid phase methodologies) of analogs to be designed easily by changing the amino acids, thereby facilitating the search for stronger and more specific HDAC inhibitors. Trapoxins (see above) are also cyclic tetrapeptides that are closely related to apicidins. However, the main difference between the 2 groups of substances is that the former bear an epoxyketone functionality rather than an alkylketone functionality, which makes the compound much less stable under physiologic conditions.

This probably means that hypermethylation and/or deacetylation and thereby inactivation of tumor suppressing genes, that act as a result of activation of the somatostatin receptor or dopamine receptor, prevents the normal cascade of events which occurs by applying somatostatin, dopamine and/or their analogs or agonists. In order to still be able to use these compounds for anti-tumor therapies despite this hypermethylation and/or deacetylation effect, very high doses of these compounds would be needed to observe any activity at all. As described herein, however, both for the somatostatin analogs and/or dopamine agonists and the demethylating agents and/or HDAC inhibitors, lower doses can be used because of the strong synergistic effect. This is advantageous, since this minimizes side effects of those compounds, which could have a negative effect on normal tissue. The synergistic effect is even enhanced by the fact that it has appeared that demethylating agents increase the expression of somatostatin and/or dopamine receptors at the surface of tumor cells.

As discussed in the introductory part, a fair number of somatostatin analogs are known in the art. These comprise derivatives of somatostatin itself (such as somatostatin-14 and somatostatin-28) or of cortistatin (cortistatin-14, cortistatin-17, cortistatin-29). Pharmaceutically preferable somatostatin analogs are octreotide, vapreotide, lanreotide, CH275, CH288, BIM 23926, BIM23206, BIM 23056, BIM 23268, BIM 23052, BIM 23244 and D Tyr(8)CYN 154806 and the recently developed non peptide agonists L 779,976, L 803,087 and L 817,818 (Olias, G et al, 2004, J Neurochem 89: 1057-1091). As dopamine agonists any of the well known amantadine, bromocriptine, cabergoline, quinagolide, lisuride, pergolide, ropinirole, pramiprexole and rasagiline can be used. Very recently somatostatin-dopamine hybrid or chimeric molecules have been synthesized, such as BIM 23A387 (Saveanu, A. et al., 2002, J. Clin. Endocrin. Metab. 87:5545-5552), BIM 23A758, BIM 23A760, BIM 23A761 and BIM 23A765 (Jaquet, P. et al., 2005, Eur. J. Endocrinol. 153: 135-141), which all show high affinity to both the somatostatin and the dopamine receptor, as well as the " universal" somatostatin ligands SOM-230 (Bruns, C. et al., 2002, Eur. J. Endocrinol. 146:707-716) and KE-108 (Reubi, J.C., et al., 2002, Eur. J. Pharmacol. 456:45-49), which bind with high affinity to most somatostatin receptor subtypes. SOM-230 and KE-108 have suitable half-life characteristics.

The above mentioned compounds may normally be administered in the way they are administered in single drug therapy. This means that they can be administered as single dose or as repeated dosages in solid or fluid form (as tablet, lozenge, granules, powder, sachet, capsule, suppository, emulsion, solution, liniment, tincture, gel, etc.), or via a slow-release formulation (in or on carriers such as lipids, liposomes or implants, in prodrug-form or in slowly metabolizing derivatives, etc.).

For the dopamine agonists this means that they may preferably administered in tablet form orally, although for the ease of combined administration with other compounds as described in the methods and composition herein also other dosage forms (gels, liquids, sprays, injection fluids, etc.) and other routes of administration (subcutaneous, intramuscular, intragluteal, intravenous, via local delivery (for example by implants), transdermal, etc.). The proteinaceous somatostatin analogues may be administered per injection. Octreotide may be given as subcutaneous injection or deep intragluteal injection (Octreotide-LAR), while lanreotide preferably may be administered as intramuscular or subcutaneous injection (lanreotide-autogel).

Administration route of HDACs depends on the sort of compound that is used, and may be, for example, oral or by injection. A similar approach is feasible for the demethylating agents discussed above.

Doses which need to be applied can be derived from doses and dose schemes which are used with the normal administration of the above drugs, although, because of the synergistic effect of the combinations of the invention, it is advised to start with lower amounts of the drug(s). Thus, for each of the above given substances, suitable dose schemes can be derived from the prior art and adapted according to the new synergistic effects as is suitable.

A typical dose for the dopamine agonist bromocriptine is 2.5-40 mg daily by oral administration (tablet). Similarly for cabergoline oral doses of 0.5-4.5 mg per week given in one or two doses is suitable. Octreotide is preferably given by injection; it can be administered in doses of 0,05-0.5 mg by subcutaneous injection every 8-12 hours or for Octreotide-LAR in doses of 10-40 mg by deep intragluteal injection once every 4 weeks. The long active lanreotide can be given by a 30 mg intramuscular injection every 1-2 weeks, or for Lanreotide-autogel in doses of 60-120 mg through a deep subcutaneous injection every 3-4 weeks. These are just non-limiting examples of the various doses and administration routes that can be applied for a few of the compounds described herein. A person skilled in the art will have the knowledge and skill to select suitable doses and administration routes for these and other compounds mentioned above.

The demethylating agent and/or HDAC inhibitor can be given simultaneously with the somatostatin analog and/or dopamine agonist, or they can be given separately. The wording 'pharmaceutical composition' as used herein can have the conventional meaning of a composition of two or more of the drugs as discussed above; however it is also meant to comprise doses for treatments wherein the drugs are administered separately, e.g. at different time intervals, but where the combination of the drugs provides the therapeutical effect. Since, especially the somatostatin analogs are given preferably as depot injections (which will be effective for several days to weeks) a simultaneous oral dosage scheme of the demethylating agent(s) and/or HDAC inhibitor(s) can be envisaged. However, these can also be given as slow release formulations, to minimize discomfort for and/or non-compliance by the patient. Preferably, the demethylating agent and/or HDAC inhibitor are given before administration of the somatostatin analog and/or dopamine agonist. In this way it is achieved that the cell machinery (receptor(s), signaling cascade, etc.) which signals and exerts the effects of somatostatin and/or dopamine are ready in functional form. The investigators have found that pre-treatment with AZA (decitabine) for one day already induces a synergistic enhanced activity with a somatostatin analog or a dopamine agonist or a somatostatin-dopamine chimeric molecule on DNA fragmentation (apoptosis) or cell proliferation.
A composition according to the invention basically comprises two components (one component selected from the group of demethylating agents consisting of 5-aza-2'-deoxycitidine (DAC, Decitabine), 5-azacytidine (5-azaC), arabinosyl-5-azacytosine (fazarabine) and dihydro-5-azacytidine (DHAC), the second component selected from the group of dopamine agonists consisting of amantadine, bromocriptine, cabergoline, quinagolide, lisuride, pergolide, ropinirole, pramiprexole, rasagiline, BIM23A387, BIM-23A758, BIM-23A760, BIM-232A761 and BIM-23A765 ). In a preferred embodiment the composition comprises three or four components. In the three component system, the composition comprises at least one demethylating agent, and at least one dopamine agonist as above, and at least one HDAC inhibitor selected from the group consisting of hydroxaminic acids, carboxylic acids, benzamides, epoxides, FK228, apicidin A, B and C. In the four component system, the composition comprises at least one demethylating agent, at least one dopamine agonist and at least one HDAC inhibitor as above, and at least one somatostatin analog selected from the group consisting of somatostatin-14, somatostatin-28, cortistatin-14, cortistatin-17, cortistatin-29, octreotide, vapreotide, lanreotide, CH275, CH-288, BIM-23056, BIM-23206, BIM-23268, BIM-23926, BIM-23052, BIM-23244, BIM-23A758, BIM-23A760, BIM-232A761, BIM-23A765, L- and D-Tyr(8)CYN154806, L-779,976, L-803,087, L-817,818, BIM23A387, SOM-320 and KE-108.
In the two or four component system, a component selected from the group of somatostatin analogs and dopamine agonists advantageously is a somatostatin-dopamine chimeric ligand, such as 6-propyl-8β-ergolinylmethylthioacetyl-D-Phe-c(Cys-Tyr-D-Trp-Lys-Abu-Cys)-Thr-NH₂, 6-propyl-8β-ergolinylmethylthioacetyl-Lys(6-propyl-88-ergolinylmethylthioacetyl)-D-Tyr-D-Tyr-c(Cys-Tyr-D-Trp-Lys-Abu-Cys)-Thr-NH₂, D-6-propyl-8β-ergolinylmethyl-thioacetyl-D-Lys(D-6-propyl-8β-ergolinyl-methylthioacetyl)-c(Cys-Tyr-D-Trp-Lys-Abu-Cys)-Thr-NH₂, 6-propyl-8β-ergolinylmethylthioacetyl-D-Phe-c(Cys-3-1-Tyr-D-Trp-Lys-Thr-Cys)-Thr-NH₂ or 6-propyl-8β-ergolinylmethylthioacetyl-Lys(6-propyl-8β-ergolinylmethylthioacetyl)-D-Tyr-D-Tyr-c(Cys-3-1-Tyr-D-Trp-Lys-Thr-Cys)-Thr-NH₂ or a universal somatostatin analog such as SOM-230 or KE-108.

In one aspect the invention also provides a pharmaceutical composition comprising 5-aza-2'-deoxycytidine and octreotide, and a pharmaceutical composition comprising 5-aza-2'-deoxycytidine and BIM-23926 or BIM-23206.

Next to a composition also a method of treatment is described herein. As indicated, such a method of treatment may comprise simultaneous or sequential administration of at least one demethylating agent or at least one HDAC inhibitor and at least one somatostatin analog or at least one dopamine agonist or at least one somatostatin-dopamine chimeric ligand or at least one universal somatostatin analog. If sequential, i.e. non-simultaneous administration is applied, it is preferred to administer the demethylating agent and/or HDAC inhibitor before administration of the other compounds. Preferably, treatment with the demethylating agent and/or HDCA inhibitor is started several days (at least one day) to maximal two weeks before the administration of the somatostatin analog or dopamine agonist.

The compositions of the invention can be used for the therapy of tumors in patients which have a tumor which expresses somatostatin and/or dopamine receptors on its surface. Many tumors have been found to comprise at least one of both receptors, and generally both receptors are expressed together. Tumors which would be suitable for therapy with the compositions of the invention comprise neuroendocrine tumors, such as pituitary adenoma, islet-cell tumor, insulinoma, carcinoid, pheochromocytoma, paragaglioma, medullary thyroid cancer; adenocarcinomas, such as breast, kidney, colon, pancreas, prostate or ovary tumor; brain tumors, such as meningioma; hematological malignancies, such as Hodgkin's and non-Hodgkin's lymphoma and acute and chronic leukemia; other malignancies like hepatocellular cancer.

Besides the advantage of combating tumor proliferation, the compositions of the invention will also prevent excess hormonal secretion from the tumours, thereby decreasing the pleiotropic effects induced by the hormone excess that are experienced by patients with these tumors.

### Example

### Methods

### Cell lines and culture conditions

Human pancreatic carcinoid BON cells were obtained from The University of Texas Medical Branch, Galveston, Texas, USA. The cells were cultured in a humidified incubator containing 5% CO₂ at 37°C. The culture medium consisted of a 1:1 mixture of Dulbecco's modified Eagle's medium (DMEM) and F12K medium, supplemented with 10% FCS, penicillin (1x105 U/l), fungizone (0.5 mg/l) and 1-glutamine (2 mmol/l). Periodically, cells were confirmed as Mycoplasma-free. Cells were harvested with trypsin EDTA 10%

### Example

### Methods

### Cell lines and culture conditions

Human pancreatic carcinoid BON cells were obtained from The University of Texas Medical Branch, Galveston, Texas, USA. The cells were cultured in a humidified incubator containing 5% CO₂ at 37°C. The culture medium consisted of a 1:1 mixture of Dulbecco's modified Eagle's medium (DMEM) and F12K medium, supplemented with 10% FCS, penicillin (1x105 U/l), fungizone (0.5 mg/l) and 1-glutamine (2 mmol/l). Periodically, cells were confirmed as Mycoplasma-free. Cells were harvested with trypsin EDTA 10% and resuspended in medium. Before plating, cells were counted microscopically using a standard hemocytometer. Trypan Blue staining was used to assess cell viability, which always exceeded 95%. Media and supplements were obtained from GIBCO Bio-cult Europe (Invitrogen, Breda, The Netherlands).

### Cell proliferation assay

After trypsinization, the cells were plated in 1 ml of medium in 24-well plates at a density of 0.4-2x104 cells/well. The plates were then placed in a 37°C, 5% CO₂ incubator. Three days later the cell culture medium was replaced with 1 ml/well medium containing 5-aza-2'-deoxycytidine at different concentrations in the dose curve response experiment (0, 10⁻⁵M, 10⁻⁶M, 10⁻⁷M, 10⁻⁸M and 10⁻⁹M) or 5-aza-2'-deoxycytidine (5x10⁻⁸M) with or without the somatostatin analog octreotide (10⁻⁸M) or the dopamine agonist cabergoline (10⁻⁸M). In experiments evaluating the combination of 5-aza-2'-deoxycytidine and octreotide or cabergoline, a pre-treatment with 5-aza-2'-deoxycytidine (5x10⁻⁸M) has been performed for 24 hours.

Plates were further incubated at 37°C and 5% CO₂. After 3 and 6 days of treatment cells were harvested for DNA measurement. Plates for 6

### Apoptosis assays (DNA fragmentation)

After trypsinization BON cells were plated in 24-well plates at a density of 1.5-2x10⁴ cells/well. These plates were placed in a 37°C, 5% CO₂ incubator. Three days later the medium was refreshed in the presence and absence (control group) of 5-aza-2'-deoxycytidine at different concentrations in the dose curve response experiment (10⁻⁵M, 10⁻⁶M, 10⁻⁷M, 10⁻⁸M and 10⁻⁹M) or 5-aza-2'-deoxycytidine (5x10⁻⁸M) with or without the somatostatin analog octreotide (10⁻⁸M) or the dopamine agonist cabergoline (10⁻⁸M). In experiments evaluating the combination of 5-aza-2'-deoxycytidine and octreotide, cabergoline or novel somatostatin analogs BIM 23206, BIM 23926 and the somatostatin-dopamine chimera BIM 23A765, a pre-treatment with 5-aza-2'-deoxycytidine (5x10⁻⁸M) has been performed for 24 hours.

Quadruplicates of each treatment were performed. After an additional incubation of 3 days, apoptosis was assessed using a commercially available ELISA kit (Cell Death Detection ELISAPlus, Roche Diagnostic GmbH, Penzberg, Germany). This assay is based on a quantitative sandwich-enzyme-immunoassay-principle, using mouse monoclonal antibodies directed against DNA and histones, respectively. This allows the specific determination of mono-and oligo-nucleosomes in the cytoplasmatic fraction of cell lysate, typical for apoptosis. The standard protocol supplied by the manufacturer was used. Briefly, the medium was carefully removed by suction, cells were incubated in 100 µl of lysis buffer (provided with the Cell Death Detection ELISAPlus assay). After lysis of the cells the solution was incubated with anti-DNA and anti-histone antibodies in a streptavidin-coated 96-well plate for 2 hours. After washing, the immunocomplex-bound peroxidase was probed with 2,2'-azino-di[3-ethylbenzthiazoline sulfonate] (a chromogenic substrate) for spectrophotometric detection at 405 nm, to quantify the amount of enzyme-immunoassay-principle, using mouse monoclonal antibodies directed against DNA and histones, respectively. This allows the specific determination of mono- and oligo-nucleosomes in the cytoplasmatic fraction of cell lysate, typical for apoptosis. The standard protocol supplied by the manufacturer was used. Briefly, the medium was carefully removed by suction, cells were incubated in 100 µl of lysis buffer (provided with the Cell Death Detection ELISAPlus assay). After lysis of the cells the solution was incubated with anti-DNA and anti-histone antibodies in a streptavidin-coated 96-well plate for 2 hours. After washing, the immunocomplex-bound peroxidase was probed with 2,2'-azino-di[3-ethylbenzthiazoline sulfonate] (a chromogenic substrate) for spectrophotometric detection at 405 nm, to quantify the amount of nucleosomes bound to the plate. Relative apoptosis was determined by a ratio of the average absorbance of the treatment wells to the average absorbance of the control wells.

### Results

### Effect of octreotide and cabergoline on cell proliferation.

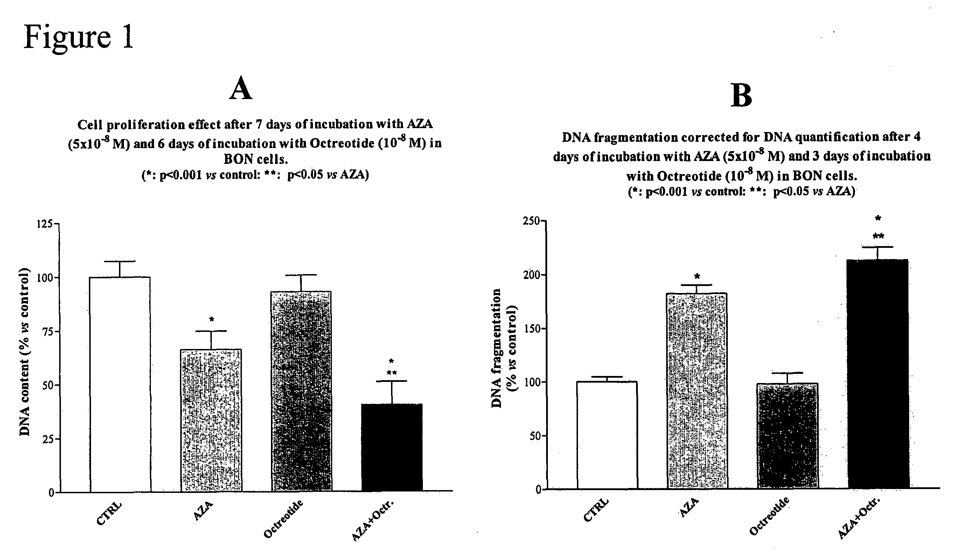

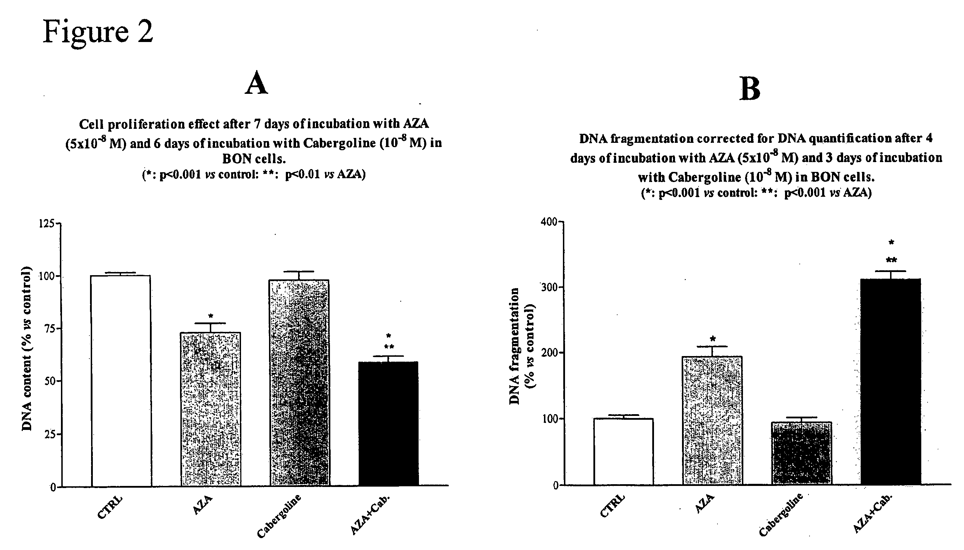

During a 3 or 6 day incubation with octreotide (10⁻⁸M) or cabergoline (10⁻⁸M), there was no statistically significant effect of both drugs on BON carcinoid cell growth, measured as the DNA content reflecting the number of cells. Figure 1A and 2A, each representing the mean ± SEM of two independent experiments, show the effects of octreotide or cabergoline after 6 days of incubation.

### Effect of 5-aza-2'-deoxycytidine on cell proliferation.

During a 3 or 6 day incubation with 5-aza-2'-deoxycytidine significantly inhibited BON carcinoid cell growth, in a time- and dose-dependent manner (data not shown). After 6 days of incubation, the IC25 value of the tumor cell growth inhibitory effect of 5-aza-2'-deoxycytidine was

### Effect of octreotide and cabergoline on apoptosis (DNA-fragmentation).

During a 3 day incubation with octreotide (10⁻⁸M) or cabergoline (10⁻⁸M), there was no statistically significant effect of both drugs on apoptosis, measured as DNA fragmentation, in BON carcinoid cells. Figure 1B and 2B, representing the mean ± SEM of two independent experiments, show the effects of octreotide or cabergoline on the induction of DNA fragmentation. A similar result was obtained for treatment with the somatostatin analogs BIM 23926 and BIM 23206 and the somatostatin-dopamine chimera BIM 23A765 alone.

### Effect of 5-aza-2'-deoxycytidine on apoptosis (DNA-fragmentation).

A 3 day incubation with 5-aza-2'-deoxycytidine significantly induced DNA fragmentation in BON carcinoid cells, in a dose-dependent manner (data not shown). After 3 days of incubation, the EC25 value of the apoptosis inducing effect of 5-aza-2'-deoxycytidine was approximately 5x10⁻⁸M. Figure 1B and 2B show that a low dose of 5-aza-2'-deoxycytidine (5x10⁻⁸M) significantly induced DNA fragmentation by approximately 100% in BON carcinoid cells (p<0.001 vs control).

### Effect of combination treatment with 5-aza-2'-deoxycytidine and octreotide or cabergoline on apoptosis (DNA-fragmentation).

Figure 1B and 2B show that the combined treatment with 5-aza-2'-deoxycytidine and octreotide (10⁻⁸M) or cabergoline (10⁻⁸M) induced a statistically significant higher induction of DNA fragmentation in BON carcinoid cells after 3 days, compared with the effect of 5-aza-2'-deoxycytidine alone (p<0.05 and p<0.001, respectively).

### Effect of combination treatment with 5-aza-2'-deoxycytidine and novel somatostatin analogs BIM 23926 and BIM 23206 on apoptosis.

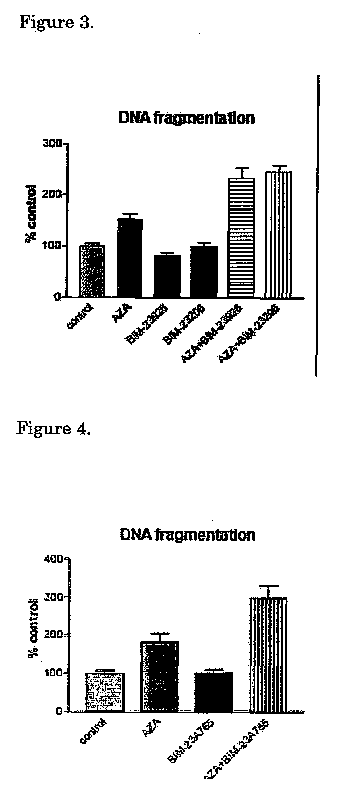

Figure 3 shows that the somatostatin analogs when administered alone have no effect on apoptosis (DNA fragmentation) of human BON carcinoid cells. However, a dramatic increase was observed from the moderate effect of AZA alone, when AZA was administered together with any of the new analogs.

### Effect of combination treatment with 5-aza-2'-deoxycytidine and the somatostatin-dopamine chimera BIM 23A765 on apoptosis.

Figure 4 shows that the somatostatin-dopamine chimera when administered alone has no effect on apoptosis (DNA fragmentation) of human BON carcinoid cells. However, a dramatic increase was observed from the moderate effect of AZA alone, when AZA was administered together with BIM 23A765.

## Claims

1. Pharmaceutical composition comprising at least one demethylating agent and at least one dopamine agonist;
wherein the at least one demethylating agent is selected from the group consisting of 5-aza-2'-deoxycitidine (DAC, Decitabine), 5-azacytidine (5-azaC), arabinosyl-5-azacytosine (fazarabine) and dihydro-5-azacytidine (DHAC); and the at least one dopamine agonist is selected from the group consisting of amantadine, bromocriptine, cabergoline, quinagolide, lisuride, pergolide, ropinirole, pramiprexole, rasagiline, BIM23A387, BIM-23A758, BIM-23A760, BIM-232A761 and BIM-23A765.

2. Pharmaceutical composition according to claim 1, wherein said composition further comprises at least one HDAC inhibitor selected from the group consisting of hydroxaminic acids, carboxylic acids, benzamides, epoxides, FK228, apicidin A, B and C.

3. Pharmaceutical composition according to claim 2, comprising at least one demethylating agent, at least one HDAC inhibitor, at least one somatostatin analog selected from the group consisting of somatostatin-14, somatostatin-28, cortistatin-14, cortistatin-17, cortistatin-29, octreotide, vapreotide, lanreotide, CH275, CH-288, BIM-23056, BIM-23206, BIM-23268, BIM-23926, BIM-23052, BIM-23244, BIM-23A758, BIM-23A760, BIM-232A761, BIM-23A765, L- and D-Tyr(8)CYN154806, L-779,976, L-803,087, L-817,818, BIM23A387, SOM-320 and KE-108, and at least one dopamine agonist.

4. Pharmaceutical composition according to claim 2, wherein said hydroxaminic acid is selected from the group consisting of trichostatin A, SAHA, NVP-LAQ-82425 and PXD-101; or
wherein said carboxylic acid is selected from the group consisting of butanoic acid, valproic acid and 4-phenoxybutanoic acid; or
wherein said benzamide is selected from the group consisting of N-acetyldinaline and MS-275; or
wherein said epoxide is selected from the group consisting of depeudicin and trapoxin A.

5. Pharmaceutical composition according to any of claim 3, wherein at least one somatostatin analog is selected from the group consisting of somatostatin-14, somatostatin-28, cortistatin-14, cortistatin-17, cortistatin-29, octreotide, vapreotide and lanreotide.

6. Pharmaceutical composition according to any of claim 3, wherein at least one somatostatin analog is selected from the group consisting of SOM-230 and KE-108.

7. Pharmaceutical composition according to any of claims 1-6, wherein the at least one dopamine agonist is selected from the group consisting of amantadine, bromocriptine, cabergoline, quinagolide, lisuride, pergolide, ropinirole, pramiprexole and rasagiline.

8. Pharmaceutical composition according to claim 1, wherein the demethylating agent is 5-aza-2'-deoxycytidine and the dopamine agonist is cabergoline.

9. Pharmaceutical composition according to claim 1, wherein the demethylating agent is 5-aza-2'-deoxycytidine and the at least one dopamine agonist is the somatostatin-dopamine chimera BIM-23A765.

10. Pharmaceutical composition comprising 5-aza-2'-deoxycytidine and octreotide.

11. Pharmaceutical composition comprising 5-aza-2'-deoxycytidine and BIM-23926 or BIM-23206.

12. A pharmaceutical composition according to any of claims 1-9 for use in the treatment of a tumor which expresses dopamine receptors on its surface.

13. A pharmaceutical composition for use according to claim 12, wherein said tumor is a neuroendocrine tumor, an adenocarcinoma, a brain tumor, a hematological malignancy, and a hepatocellular cancer.

14. A pharmaceutical composition for use according to claim 13, wherein said neuroendocrine tumor is selected from the group consisting of pituitary adenoma, islet-cell tumor, insulinoma, carcinoid, pheochromocytoma, paragaglioma, medullary thyroid cancer; or
wherein said adenocarcinoma is selected from the group consisting of breast, kidney, colon, pancreas, prostate or ovary tumor; or
wherein said brain tumor is a meningioma; or
wherein said hematological malignancy is selected from the group consisting of Hodgkin's and non-Hodgkin's lymphoma and acute and chronic leukemia.

15. A pharmaceutical composition as defined in any one of claims 1 to 11 for use in the treatment of a tumor.

## Patentansprüche

1. Pharmazeutische Zusammensetzung, umfassend mindestens ein Demethylierungsmittel und mindestens ein Dopaminagonist, worin das mindestens eine Demethylierungsmittel gewählt ist aus der Gruppe bestehend aus 5-Aza-2'-deoxycitidin (DAC, Decitabin), 5-Azacytidin (5-azaC), Arabinosyl-5-azacytosin (Fazarabin) und Dihydro-5-azacytidin (DHAC) und der mindestens eine Dopaminagonist gewählt ist aus der Gruppe bestehend aus Amantadin, Bromocriptin, Cabergolin, Quinagolid, Lisurid, Pergolid, Ropinirol, Pramiprexol, Rasagilin, BIM23A387, BIM-23A758, BIM-23A760, BIM-232A761 und BIM-23A765.

2. Pharmazeutische Zusammensetzung nach Anspruch 1, worin die Zusammensetzung weiter mindestens einen HDAC-Inhibitor umfasst, gewählt aus der Gruppe bestehend aus Hydroxaminsäuren, Carbonsäuren, Benzamiden, Epoxiden, FK228, Apicidin A, B und C.

3. Pharmazeutische Zusammensetzung nach Anspruch 2, umfassend mindestens ein Demethylierungsmittel, mindestens einen HDAC-Inhibitor, mindestens ein Somatostatin-Analogon, gewählt aus der Gruppe bestehend aus Somatostatin-14, Somatostatin-28, Cortistatin-14, Cortistatin-17, Cortistatin-29, Octreotid, Vapreotid, Lanreotid, CH275, CH-288, BIM-23056, BIM-23206, BIM-23268, BIM-23926, BIM-23052, BIM-23244, BIM-23A758, BIM-23A760, BIM-232A761, BIM-23A765, L- und D-Tyr(8)CYN154806, L-799,976, L-803,087, L-817,818, BIM23A387, SOM-320 und KE-108 und mindestens einen Dopaminagonisten.

4. Pharmazeutische Zusammensetzung nach Anspruch 2, worin die Hydroxaminsäure gewählt ist aus der Gruppe bestehend aus Trichostatin A, SAHA, NVP-LAQ-82425 und PXD-101 oder
worin die Carbonsäure gewählt ist aus der Gruppe bestehend aus Butansäure, Valproinsäure und 4-Phenoxybutansäure oder
worin das Benzamid gewählt ist aus der Gruppe bestehend aus N-Acetyldinalin und MS-275 oder
worin das Epoxid gewählt ist aus der Gruppe bestehend aus Depeudicin und Trapoxin A.

5. Pharmazeutische Zusammensetzung nach Anspruch 3, worin das mindestens eine Somatostatin-Analogon gewählt ist aus der Gruppe bestehend aus Somatostatin-14, Somatostatin-28, Cortistatin-14, Cortistatin-17, Cortistatin-29, Octreotid, Vapreotid und Lanreotid.

6. Pharmazeutische Zusammensetzung nach Anspruch 3, worin das mindestens eine Somatostatin-Analogon gewählt ist aus der Gruppe bestehend aus SOM-230 und KE-108.

7. Pharmazeutische Zusammensetzung nach einem der Ansprüche 1 - 6, worin der mindestens eine Dopaminagonist gewählt ist aus der Gruppe bestehend aus Amantadin, Bromocriptin, Cabergolin, Quinagolid, Lisurid, Pergolid, Ropinirol, Pramiprexol und Rasagilin.

8. Pharmazeutische Zusammensetzung nach Anspruch 1, worin das Demethylierungsmittel 5-Aza-2'-deoxycytidin ist und der Dopaminagonist Cabergolin ist.

9. Pharmazeutische Zusammensetzung nach Anspruch 1, worin das Demethylierungsmittel 5-Aza-2'-deoxycytidin ist und der mindestens eine Dopaminagonist die Somatostatin-Dopamin-Chimäre BIM-23A765 ist.

10. Pharmazeutische Zusammensetzung umfassend 5-Aza-2'-deoxycytidin und Octreotid.

11. Pharmazeutische Zusammensetzung umfassend 5-Aza-2'-deoxycytidin und BIM-23926 oder BIM-23206.

12. Pharmazeutische Zusammensetzung nach einem der Ansprüche 1 - 9 zur Verwendung in der Behandlung eines Tumors, der auf seiner Oberfläche Dopaminrezeptoren exprimiert.

13. Pharmazeutische Zusammensetzung zur Verwendung nach Anspruch 12, worin der Tumor ein neuroendokriner Tumor, ein Adenokarzinom, ein Gehirntumor, eine hämatologische Malignität und ein Leberzellkrebs ist.

14. Pharmazeutische Zusammensetzung zur Verwendung nach Anspruch 13, worin der neuroendokrine Tumor gewählt ist aus der Gruppe bestehend aus Hypophysenadenom, Inselzelltumor, Insulinom, Karzinoid, Phäochromozytom, Paragagliom, medullärer Schilddrüsenkrebs oder
worin das Adenokarzinom gewählt ist aus der Gruppe bestehend aus Brust-, Nieren-, Darm-, Pankreas-, Prostata- oder Eierstocktumor oder
worin der Gehirntumor ein Meningiom ist oder
worin die hämatologische Malignität gewählt ist aus der Gruppe bestehend aus Hodgkin- oder Nicht-Hodgkin-Lymphom und akute und chronische Leukämie.

15. Pharmazeutische Zusammensetzung nach einem der Ansprüche 1 - 11 zur Verwendung in der Behandlung eines Tumors.

## Revendications

1. Composition pharmaceutique comprenant au moins un agent de déméthylation et au moins un agoniste de la dopamine ;
dans laquelle le au moins un agent de déméthylation est choisi dans le groupe constitué par la 5-aza-2'-désoxycitidine (DAC, Decitabine), 5-azacytidine (5-azaC), l'arabinosyl-5-azacytosine (fazarabine) et la dihydro-5-azacytidine (DHAC) ; et le au moins un agent agoniste de la dopamine est choisi dans le groupe constitué par l'amantadine, la bromocriptine, la cabergoline, le quinagolide, le lisuride, le pergolide, le ropinirole, le pramiprexole, la rasagiline, BIM23A387, BIM-23A758, BIM-23A760, BIM-232A761 et BIM-23A765.

2. Composition pharmaceutique selon la revendication 1, dans laquelle ladite composition comprend en outre au moins un inhibiteur de HDAC choisi dans le groupe constitué par les acides hydroxaminiques, les acides carboxyliques, les benzamides, les époxydes, FK228, l'apicidine A, B, et C.

3. Composition pharmaceutique selon la revendication 2, comprenant au moins un agent de déméthylation, au moins un inhibiteur de HDAC, au moins un analogue de somatostatine choisi dans le groupe constitué par la somatostatine-14, la somatostatine-28, la cortistatine-14, la cortistatine-17, cortistatine-29, l'octréotide, le vapréotide, le lanréotide, CH275, CH-288, BIM-23056, BIM-23206, BIM-23268, BIM-23926, BIM-23052, BIM-23244, BIM-23A758, BIM-23A760, BIM-232A761, BIM-23A765, L- et D-Tyr(8)CYN154806, L-779,976, L-803,087, L-817,818, BIM23A387, SOM-320 et KE-108, et au moins un agoniste de dopamine.

4. Composition pharmaceutique selon la revendication 2, dans laquelle ledit acide hydroxaminique est choisi dans le groupe constitué par la trichostatine A, SAHA, NVP-LAQ-82425 et PXD-101 ; ou
dans laquelle ledit acide carboxylique est choisi dans le groupe constitué par l'acide butanoïque, l'acide valproïque et l'acide 4-phénoxybutanoïque ; ou
dans laquelle ledit benzamide est choisi dans le groupe constitué par la N-acétyldinaline et MS-275 ; ou
dans laquelle ledit époxyde est choisi dans le groupe constitué par la dépeudicine et la trapoxine A.

5. Composition pharmaceutique selon la revendication 3, dans laquelle au moins un analogue de somatostatine est choisi dans le groupe constitué par la somatostatine-14, la somatostatine-28, la cortistatine-14, la cortistatine-17, cortistatine-29, l'octréotide, le vapréotide et le lanréotide.

6. Composition pharmaceutique selon la revendication 3, dans laquelle au moins un analogue de somatostatine est choisi dans le groupe constitué par SOM-230 et KE-108.

7. Composition pharmaceutique selon l'une quelconque des revendications 1 à 6, dans laquelle le au moins un agoniste de dopamine est choisi dans le groupe constitué par l'amantadine, la bromocriptine, la cabergoline, le quinagolide, le lisuride, le pergolide, le ropinirole, le pramiprexole et la rasagiline.

8. Composition pharmaceutique selon la revendication 1, dans laquelle l'agent de déméthylation est la 5-aza-2'-désoxycytidine et l'agoniste de la dopamine est la cabergoline.

9. Composition pharmaceutique selon la revendication 1, dans laquelle l'agent de déméthylation est la 5-aza-2'-désoxycytidine et le au moins un agoniste de la dopamine est la chimère de somatostatine-dopamine BIM-23A765.

10. Composition pharmaceutique comprenant la 5-aza-2'-désoxycytidine et l'octréotide.

11. Composition pharmaceutique comprenant la 5-aza-2'-désoxycytidine et BIM-23926 ou BIM-23206.

12. Composition pharmaceutique selon l'une quelconque des revendications 1 à 9 pour l'utilisation dans le traitement d'une tumeur qui exprime les récepteurs de la dopamine sur sa surface.

13. Composition pharmaceutique pour l'utilisation selon la revendication 12, dans laquelle ladite tumeur est une tumeur neuroendocrinienne, un adénocarcinome, une tumeur du cerveau, une malignité hématologique, et un cancer hépatocellulaire.

14. Composition pharmaceutique pour l'utilisation selon la revendication 13, dans laquelle ladite tumeur neuroendocrinienne est choisie dans le groupe constitué par l'adénome pituitaire, la tumeur des cellules-îlots, l'insulinome, le carcinoïde, le phéochromocytome, le paragagliome, le cancer de la thyroïde médullaire , ou
dans laquelle ledit adénocarcinome est choisi dans le groupe constitué par la tumeur du sein, du rein, du colon, du pancréas, de la prostate ou des ovaires ; ou
dans laquelle ladite tumeur du cerveau est un méningiome ; ou
dans laquelle ladite malignité hématologique est choisie dans le groupe constitué par le lymphome hodgkinien et non hodgkinien et la leucémie aiguë et chronique.

15. Composition pharmaceutique selon l'une quelconque des revendications 1 à 11 pour l'utilisation dans le traitement d'une tumeur.
